# EUROPEAN PATENT APPLICATION

(11) **EP 4 692 305 A1**
(43) Date of publication of application: **11.02.2026**
(21) Application number: 24193978.4
(22) Date of filing: 09.08.2024
(51) Int. Cl.: C12M 3/06, C12M 1/34, C12M 1/36

(54) **METHOD FOR CONTROLLING PRESSURE AND DISSOLVED GAS COMPOSITION AND/OR PH OF A VOLUME OF LIQUID CELL CULTURE MEDIUM CONTAINED IN AT LEAST ONE CLOSED CHAMBER WHICH COMPRISES A CULTURED BIOLOGICAL ENTITY**

(71) Applicant: Cherry Biotech SAS, 93100 Montreuil (FR)
(72) Inventor: DAWSON, Harry, 93100 Montreuil (FR); HOMS CORBERA, Antoni, 93100 Montreuil (FR)
(74) Representative: Vidon Brevets & Stratégie

(57) **Abstract**

The invention concerns a method for controlling pressure and dissolved gas concentration and/or pH of a volume of liquid cell culture medium (VL) contained in at least one closed chamber (1) which comprises a cultured biological entity (3) which is composed of at least one cell. The method comprises a step of determining a local pressure of the volume of liquid cell culture medium (VL) applied to said cultured biological entity (3) based on measurements of an internal pressure inside said at least closed chamber (1), a dissolved gas(es) concentration and/or pH of said volume of liquid cell culture medium (VL), and adjusting, by a controller (4), the flow rate of the moving fluid (2) within said volume of liquid cell culture medium (VL) resulting from the injection and/or the extraction to control said local pressure regarding said measurements.

## Description

### Technical field

The invention concerns the field of in vitro culture of cells, tissues or organoids on culture plates and especially on multi-well plates.

In particular, the invention relates to the control of the environmental conditions of *in vitro* culture within a confined space such as cell culture plates, or in fluidic devices.

More particularly, the invention relates to a method for controlled dissolved gas(es) concentration and/or pH of a perfused liquid cell culture medium and a cell culture system configured to implement the method for *in vitro* cell culture.

More specifically, the invention concerns a method for controlling pressure and dissolved gas concentration and/or pH of a volume of liquid cell culture medium contained in at least one closed chamber which comprises a cultured biological entity and a system configured to implement the method for *in vitro* cell culture.

### Previous technique

*In vitro* cultures of cells, or of cellular assembly such as tissue culture, of spheroids or even more recently of organoids, i.e. of three-dimensional multicellular structures which reproduce *in vitro* the micro-anatomy or physiology of an organ, are today increasingly used as a tool for the toxicological evaluation of substances, in particular pharmacological or cosmetic, or biotherapies, such as those based on immune cells.

These *in vitro* models are used, particularly in pharmaceutical research, because they represent a feasible alternative to *in vivo* models, i.e. to animal experimentation, against which both economic and ethical pressures appear at the international level.

Thus, to be able to carry out controlled biological experiments using these *in vitro* models and to analyze therapeutic compounds, cosmetics or potentially dangerous substances, it is essential to automate the maintenance of cell culture parameters and the induction of selective environmental changes in confined cellular culture environments.

Microfluidic cell culture, understood as the cultivation of cells in chambers, or reservoirs, connected and fed by microchannels characterized by fluidic volumes between 1pL and 100mL, is an important technology for drug screening, tissue culture, detection of toxicity and biological research. The approach improves cell culture conditions, permits multiple cell types to communicate, as well as better control of chemical and physical mechanisms such as diffusion and shear-stress. Moreover, it provides better quality of better quality experimental data due to increased biophysical parameter control, meaning increased reproducibility, while economizing reagent consumption and therefore reducing costs.

However traditional two-dimensional cultures are often over simplistic, lacking the complexity and intercellular communication of *in vivo* models. Mechanical forces, often neglected in these models, play an essential role on the behavior of biological entities by activating downstream signaling pathways through a process termed mechanotransduction.

As an example of expression altered by mechanotransduction, cellular responses to chemical and mechanical stimuli are associated with cancer progression. The physical context of the tumor impacts the cell phenotype transforming normal tissue and driving progression of primary tumor invasion, dissemination, and metastasis. In recent years, mechanotransduction has been applied to more complex *in vitro* models that better recapitulate human and animal physiology. Associated recent research has shown the importance of incorporating pressure control on such models to enhance regulation to promote phenotype changes and to reduce apoptosis levels in cell and biological tissues.

On the other hand, the plasticity (i.e. deformability of cell phenotypes and cell contained structures) and motility of cancer cells (i.e. how cells move as a response to environmental cues) can also be used to estimate the metastatic and aggressiveness potential of a given cancer cell or tissue. This has many applications in personalized diagnostics leading to tailored patient therapeutic approaches. For example, as disclosed in WO2013041803, pressure application on biological entities linked to the readout of morphological and biochemical parameters has been shown as an effective method to assess those properties on cells and cell contained structures.

As such, the pressure integration with *in vitro* models has remained a priority for both physical biomarker exploration but also providing physiological levels of pressure, an important factor to recreate in *vivo* micro-environments for such models.

For example, WO2013095834A1 discloses an approach to mimic *in vivo* physiological pressure using hydraulic presses and pistons on a closed vessel containing a biological entity. Limitations of this approach includes compromising the sterility of the system. Although this approach provides a method to create pressure gradients and cycles, media cannot be renewed and the dependence of pH or dissolved oxygen notably with pressure is not managed. These limitations make the approach non-suitable for long term cell culture.

In another example, US4851354A uses hydrostatic pressure by means of an airtight well having an optically transparent base and removable cap for local pressure control through pressurized media. The system remains highly complex for cell seeding and model development and lacks standardization. Also for long term cell culture, pressure must be removed over extended periods in order to renew the cell media within the system which reduces the physiological similarity of the in-vivo model. As with the previous example, pH or DO are not controlled during pressure changes.

There is therefore a need for a much more versatile technique and apparatus adaptable to, but not limited to, the standard of multi-well culture plates comprising at least one closed chamber for a cultured biological entity, such as tissue, spheroids, organoids or cells, confinement in highly controlled cell culture environments in the closed chamber, in terms of temperature, pressure applied by a volume of liquid cell culture medium to the cultured biological entity, gas composition of the volume of liquid cell culture medium and fluid displacement within the volume of liquid cell culture medium. Additionally, there is a need for detecting the changes in dissolved gas concentration or the pH of the volume of liquid cell culture medium to compensate these changes, for example resulting from Henry's law of gas dissolution.

### Disclosure of the Invention

The invention fulfils this need by proposing a method for controlling pressure and dissolved gas concentration and/or pH of a volume of liquid cell culture medium contained in at least one closed chamber which comprises a cultured biological entity composed of at least one cell. The method comprises the following steps:
- setting a predetermined local pressure and a predetermined dissolved gas concentration and/or a predetermined pH to be satisfied by the volume of liquid cell culture medium as a function of the cultured biological entity,
- injecting and/or extracting a fluid at a flow rate into and/or from said volume of liquid cell culture medium so that the fluid moving within said volume of liquid cell culture medium resulting from the injection and/or the extraction involves a change in a local pressure of the volume of liquid cell culture medium (VL) applied to said cultured biological entity inside said at least one closed chamber and a change in dissolved gas concentration and/or pH of the volume of liquid cell culture medium,
- measuring an internal pressure inside said at least one closed chamber, and a dissolved gas concentration and/or a pH of the volume of liquid cell culture medium by at least a pressure sensor, and a dissolved gas concentration sensor and/or a pH sensor,
- determining the local pressure at least on the basis of a measured pressure obtained by the pressure sensor,
- adjusting, by a controller, at least the flow rate of the injected fluid and/or the extracted fluid and the internal pressure so that the determined local pressure and the measured dissolved gas concentration and/or the measured pH of the volume of liquid cell culture medium correspond to the predetermined local pressure, and the predetermined dissolved gas concentration and/or the predetermined pH of the volume of liquid cell culture medium respectively,
during the step of adjusting, the flow rate of the injected fluid and/or the extracted fluid is less than or equal to 20 ml/min and the internal pressure is less than or equal to 10 bar.

Advantageously, the method according to the invention makes it possible to maintain an ideal environment for cell culture in a closed environment that totally isolates any biological entities such as: cell cultures, spheroids, tissues, organoids, cells in extracellular matrixes, cells in hydrogels, etc. from the external environment.

More particularly, the method allows to precisely control the local pressure of the volume of liquid cell culture medium applied to the cultured biological entity, and the dissolved gas concentration and/or the pH of the volume of liquid cell culture medium to better mimic *in vivo* microphysiological conditions.

In addition, the method allows to mimic *in vivo* microphysiological conditions by controlling, on the one end, the flow rate of the injected and/or extracted fluid into and/or from a closed environment, in particular the at least one closed chamber, and on the other end, the internal pressure, while eventually simultaneously maintaining other critical chemical and physical parameters of the volume of liquid cell culture medium.

By "fluid", we mean in the following description a liquid, a liquid mixture, a gas, a gas mixture, or a mixture comprising at least one liquid and at least one gas. The fluid can be any liquid or gas needed for biological experiments. Throughout the description the liquid is a cell culture medium suitable for culturing biological entities.

By "gas composition" we mean in the following description, the nature and quantity of the gas. For example, when the fluid injected is a gas mixture, the composition comprises the different types of gas in the mixture and their respective quantities (i.e., concentration of each gas and/or percentage of each gas contain in the total gas flow) within the total injected gas that moves inside the closed chamber.

By "closed chamber" we mean in the following description several possible material arrangements such as a closed microfluidic channel of a microfluidic device or a reservoir integrated in a microfluidic device or sealed by a microfluidic device, thus forming a closed chamber of a fluidic device or fluidic assembly. The reservoir can be integrated in a microfluidic channel (for example by forming a space to act as a reservoir using 3D printing), an isolated reservoir (for example petri dish or a single well of a multi-well cell culture plate) or several reservoirs mounted in parallel and/or in series (for example several well of a multi-well cell culture plate). The closed chamber is a hermetically sealed environment in which biological entities can be cultured and studied.

Thus, it is possible to cultivate biological samples within the at least one closed chamber thanks to a continuous or discontinuous controlled renewal of the culture medium, in particular its nutrients. This controlled renewal is ensured by the injection and/or the extraction of a fluid into and/or from the volume of liquid cell culture medium contained in at least one closed chamber without the culture medium coming into contact with any external environment, other than exchanging the fluid by its injection and/or its extraction.

Furthermore, the method according to the invention allows to precisely control the fluid that moves within the volume of liquid cell culture medium in order to notably allow the selective application of the local pressure that the volume of liquid cell culture medium applied to the cultured biological entity. This control is achieved thanks to at least the pressure sensor that, for example, can be within the controller, and/or within the at least one closed chamber.

Moreover, the method according to the invention makes it possible to characterize the effect of an application of pressure on a specific liquid cell culture medium and/or on a specific biological entity, in particular by determining the local pressure, which is made possible by measuring the said concentration of dissolved gas and/or by measuring the pH of the volume of liquid cell culture medium.

Thus, this method provides physiologically relevant pressure with cell media conditioning compensation for long-term cell culture studies.

This method allows to easily correct, preferably in real-time, the cell culture medium inside the closed chamber by injecting and/or extracting the fluid while controlling its flow rate and the internal pressure.

Hence, this method allows to control, preferably dynamically, the pressure applied by the volume of liquid cell culture medium to the cultured biological entity and the dissolved gas concentration and/or the pH of the volume of liquid cell culture medium at least by adjusting the fluid that it is injected and/or extracted into or from the closed chamber.

This method can be adapted to a single closed chamber or to several closed chambers, for example in a multi-well plate, using fluidic adapters developed to facilitate the implementation of this method using several closed chambers with various fluidic connections. Thus, a single closed chamber or several connected closed chambers can be used using the multi-well plate and the fluidic adapter.

This method allows to use simple pressure cycles to achieve and allow pressure values to be controlled, the range of pressure application remaining considerably high.

Finally, with this method, the dissolved gas concentration and/or the pH of the volume of liquid cell culture medium can be controlled. This method is compatible with standard culturing methods.

Preferably, the fluid is injected and/or extracted using a computer that controls the controller.

Further, the dissolved gas(es) concentration and/or the pH is then measured by sensor(s) and the data transmitted to the controller that, preferably, display it on a human-machine interface or alternatively process it. Thus, it is possible to know the gas(es) concentration (i.e., nature of the gas(es) and concentration or percentage of each gas in a total gas flow) of the liquid cell culture medium in real time and thus make adjustment(s) if necessary.

The method according to the invention enables a cell culture, a tissue or any other living matter to be maintained in a living condition by a precise control of the vital biological parameters such as the pressure of the liquid cell culture medium and the gas composition of the liquid cell culture medium and/or pH, to prevent viability from being compromised or to achieve specific cell or biological entities phenotypes.

The method according to the invention can be used for physical biomarker physiological and pathophysiological exploration with in-vitro models.

The method can be implemented on a system that comprises a plurality of closed chambers that are mounted in series and/or in parallel.

According to a particular aspect of the invention, the method further comprises a step of controlling a composition of the injected fluid to readily alter the dissolved gas concentration and/or the pH of the volume of liquid cell culture medium for facilitating the correspondence of the step of adjusting between the measured dissolved gas concentration and/or the measured pH and the predetermined dissolved gas concentration and/or the predetermined pH, respectively. For example, this step of controlling can be carried out by injecting a specific fluid directly inside the closed chamber for altering the volume of liquid cell culture medium. In another example, this step of controlling can be carried out by conditioning a fluid in a reservoir on the basis on the determined local pressure and on the measured dissolved gas concentration and/or the measured pH.

Thus, it is possible to move, or displace, the fluid into and/or from the volume of liquid cell culture medium with a specific composition and/or pH. More particularly, the fluid that flows is totally isolated from the external environment, thus guaranteeing the integrity of the biological entity cultured.

It is thus possible to regularly control the pressure applied by the volume of liquid cell culture medium on the cultured biological entity and to regularly control the composition and/or the pH of the volume of liquid cell culture medium. Thus, composition, nutrients, pH etc. of the volume of liquid cell culture medium are controlled to approximate in vivo conditions as closely as possible.

According to a particular aspect of the invention, said step of controlling the composition and/or the pH of the injected fluid uses a feed-back loop automated system based on a mathematical algorithm and/or an artificial intelligence module.

Hence, the method according to the invention allows to adjust in real time, and in function of the internal pressure, and in an automated manner the gas composition and/or pH of the volume of liquid cell culture medium based on the determined local pressure.

More particularly, the determined local pressure, the measurements of the dissolved gas concentration and/or the pH are transmitted to the controller. The controller can then process the received data and compare it to the predetermined local pressure and predetermined dissolved gas concentration and/ predetermined pH value to be obtain (for example specific to the biological entity in cultivation or to the biological experiment implemented). For this, the controller uses a feedback loop based for example on a mathematical algorithm and/or an artificial intelligence module. In order to have a precise pressure adapted to the cultured biological entity, the controller also takes into account that fluid flow rates should be less than or equal to 20 ml/min and local pressures should be less than or equal to 10 bar within some of the microchannels of a microfluidic device, fluidic device or fluidic assembly.

Thus, the controller can calculate the injection and/or the extraction flow rate of the fluid that is injected and/or extracted to and/or from the volume of liquid cell culture medium and the internal pressure to be applied to achieve the predetermined local pressure and dissolved gas concentration and/or pH of the volume of the liquid cell culture medium.

For this, in one embodiment, the control module then transmits a command to adjust the gas(es) composition and/or pH in real time to the different flow controllers which adjust the gas(es) and/or liquid injection flow rate and/or pressure, iteratively or not, to reach the predetermine gas concentration and/or pH, fluid flow rate and local pressure.

According to a particular aspect of the invention, said step of adjusting the flow rate of the injected fluid and/or the extracted fluid and the internal pressure uses a feed-back loop automated system based on a mathematical algorithm and/or an artificial intelligence element considering the determined local pressure and the measured dissolved gas concentration and/or the measured pH of the volume of liquid cell culture medium.

This allows to compensate in real-time, and without human intervention, the effects of the pressure of the volume of liquid cell culture medium on the cultured biological entity, and of the dissolved gas concentration and/or the pH of the volume of liquid cell culture medium.

According to a particular aspect of the invention, wherein said method further comprises a step of determining a physiological and/or a pathophysiological condition of the cultured biological entity which presents as a result of a change in the phenotype of the cultured biological entity.

For example, the change in the phenotype of the cultured biological entity is a biochemical change or a morphological change.

Preferably, the at least one closed chamber is transparent to enable the evolution of the cultured biological entity to be observed, for example using an image sensor, to easily detect the biochemical change or the morphological change.

In one embodiment, the change in the phenotype is further altered by applying at least one external therapeutic agent providing the possibility for therapeutic testing under pre-defined conditions inside the at least one closed chamber. The difference between the change in the phenotype before and after the alteration produced by the at least one external therapeutic agent is used to determine the suitability of at least one external therapeutic agent for therapeutic purposes.

For example, as a therapeutic agent, we can cite drug, RNA, or CAR-T cells.

According to a particular aspect of the invention, wherein when the cultured biological entity is composed of at least one cancer cell, the step of determining a physiological and/or a pathophysiological condition of the cultured biological entity corresponds to a step of determining a metastatic potential of said at least one cancer cell. This makes it possible to determine the aggressiveness of the disease using a low-resource method designed to identify biomarkers with a view to establishing disease profiles.

According to a particular aspect of the invention, said method further comprises a step of measuring a temperature inside said at least one closed chamber by at least a temperature sensor. This allows the temperature inside the local chamber to be known and corrected if necessary, using heating means adapted to heat at least said one closed chamber. Hence, this makes it possible to determine and control the local pressure with greater accuracy, since it is determined on the basis of the internal pressure and temperature inside the closed chamber, and the dissolved gas concentration and/or the pH of the volume of liquid cell culture medium.

In fact, it should be noted that a change in temperature within the closed chamber also changes the pressure that the volume of liquid cell culture medium applies to the cultured biological entity. This is why it is necessary to accurately measure such a temperature in order to better control the pressure applied to the cultured biological entity, all the more so when the temperature rises as a result of heating, for example.

Furthermore, measurements of the temperature and the internal pressure inside the closed chamber can be utilized to determine the local pressure and to predict, based on an algorithmic prediction, the dissolved gas composition and/or the pH of the volume of liquid cell culture medium.

According to a particular aspect of the invention, said at least one injected fluid is chosen from among O₂, CO₂, N₂ or their mixtures.

According to a particular aspect of the invention, the cell culture system comprises a plurality of closed chambers, the step of measuring the internal pressure inside said each closed chamber corresponds to a step of calculating an average internal pressure based on the measured internal pressure inside each closed chamber, and the measured dissolved gas concentration and/or the measured pH of the volumes of liquid cell culture medium by at least pressure sensors, and dissolved gas concentration sensors and/or pH sensors.

The invention also relates to a cell culture system. The cell culture system implements the method described previously. The cell culture system comprises:
- at least one closed chamber comprising a volume of liquid cell culture medium and containing a cultured biological entity, said at least one closed chamber comprising, for injecting and/or extracting a fluid into and/or from the volume of liquid cell culture medium, an inlet side through which the fluid is injected and an outlet side through which the fluid is extracted to control the local pressure and dissolved gas concentration and/or pH of the volume of liquid cell culture medium,
- a microfluidic device comprising:
   ∘ at least two microfluidic circuits:
      ▪ a first of said at least two microfluidic circuits comprising at least one microfluidic channel configured to be connected to the inlet side of said at least one closed chamber, and
      ▪ a second of said at least two microfluidic circuits comprising at least one microfluidic channel configured to be connected to the outlet side of said at least one closed chamber,
- a controller connected to said at least one microfluidic channel of the first or the second of said at least two microfluidic circuits, the controller being configured to adjust the flow rate of the injected and/or extracted fluid and an internal pressure inside said at least one closed chamber,
- a pressure sensor configured to measure the internal pressure in said at least one closed chamber and to transmit the measured internal pressure to the controller, the pressure sensor being connected to said at least one microfluidic channel of the first or the second of said at least two microfluidic circuits or being connected to the interior of said at least one closed chamber,
- a dissolved gas concentration sensor and/or a pH sensor configured to measure a dissolved gas concentration and/or an pH in said at least one closed chamber and to transmit the measured dissolved gas concentration and/or the measured pH to the controller, the dissolved gas concentration and/or the pH sensors being connected to said at least one microfluidic channel of the first or the second of said at least two microfluidic circuits or being connected to the inside of said at least one closed chamber.

According to a particular aspect of the invention, the cell culture system further comprises a pressure-release valve actuated in synchronization with said controller, the pressure-release valve being connected to the second of said at least two microfluidic circuits when the pressure sensor is connected to the first of said at least two microfluidic circuits.

According to a particular aspect of the invention, wherein the pressure sensor is contained inside the controller when the controller is connected to said at least one microfluidic channel of the first of said at least two microfluidic circuits.

According to a particular aspect of the invention, the controller is connected to said at least one microfluidic channel of the first of said at least two microfluidic circuits, the pressure sensor and the pressure-release valve are connected to the second of said at least two microfluidic circuits.

The adjustment of the fluid flow rate and the internal pressure inside the closed chamber by using the controller and pressure-release valve in synchronization are advantageous due to the fact that the adjustment can be carried out in real-time. In particular, cyclic minimal opening and closing of the pressure-release valve can be used in order enable media perfusion of the cultured biological entity and to prevent retaining the non-physiological static nature of the cultured biological entity.

According to a particular aspect of the invention, the dissolved gas concentration sensor and/or the pH sensor are connected to said at least one closed chamber.

The sensors can be external to the at least one closed chamber or can be integrated in the at least one closed chamber. For example, these sensors are optical or electrochemical sensors.

According to a particular aspect of the invention, when the pH and/or the dissolved gas concentration sensors are placed upstream, namely between the controller connected to said at least one microfluidic channel of the first of said at least two microfluidic circuits and said at least one closed chamber, the cell culture system further comprises a complementary pH sensor and/or a complementary dissolved gas concentration sensor placed downstream, namely connected to said at least one microfluidic channel of the second of said at least two microfluidic circuits.

These complementary sensors allow to have sensors both at the inlet side and the outlet side of the at least one closed chamber in order to detect the local conditioning of the liquid cell culture medium prior and post local pressure application.

According to another aspect of the invention, the cell culture system further comprises a temperature sensor configured to measure a temperature in said at least one closed chamber and to transmit the measured temperature to the controller, the temperature sensor being connected to said at least one microfluidic channel of the first or the second of said at least two microfluidic circuits or inside said at least one closed chamber.

According to another aspect of the invention, the temperature sensor is connected to a control unit itself connected to heating means configured to modify the temperature inside said at least one closed chamber as a function of the measured temperature.

According to another aspect of the invention, said microfluidic circuits connect the volume of liquid cell culture medium contained in said at least one closed chamber to another volume of liquid cell culture medium contained in at least one other closed chamber, said volume of liquid cell culture medium being successively displaced by said at least one controller from said at least one closed chamber to said at least one other closed chamber through said microfluidic circuits.

### List of figures

Other purposes, characteristics and advantages of the invention will emerge more clearly upon reading the following description of a particular embodiment, provided as a simple non-restrictive example, in relation to the figures, among which:
**[****fig. 1]:** Figure 1 illustrates a view of a fluidic assembly of a multi-well cell culture plate with a cell culture system according to the invention.
**[****fig. 2]:** Figure 2 presents a diagram of an example of an embodiment of a cell culture system suitable for implementing the method according to the invention.
**[****fig. 3]:** Figure 3 presents a diagram of an example of an embodiment of a cell culture system suitable for implementing the method according to the invention.
**[****fig. 4]:** Figure 4 presents a diagram of an example of an embodiment of a cell culture system suitable for implementing the method according to the invention.
**[****fig. 5]:** Figure 5 presents a diagram of an example of an embodiment of a cell culture system suitable for implementing the method according to the invention.

### Detailed description of the embodiments of the invention

The general principle of the invention consists in confining and controlling in a temporal and spatial manner the chemical, biochemical and physical properties, such as the dissolved gas concentration, pH, pressure or local temperatures, of biological environments within devices, such as for example bioreactors, fluidic or microfluidic devices, or multi-well cell culture plates, also called multi-well culture plates. More particularly, the invention seeks to allow a control, preferably an automatic control, of the cell culture conditions using a specially designed device integrated into a cell culture system.

In particular, the invention provides an effective way to maintain an ideal environment, for cell culture system comprising fluidic or microfluidic device, in at least one closed chamber that totally isolates any biological entities and to precisely control the local pressure of the volume of liquid cell culture medium VL applied to the biological entities to better mimic *in vivo* microphysiological conditions.

The liquid cell culture medium can be constituted by liquid or by a mixture containing liquid and gas. It should be noted that in the following description, by volume of liquid cell culture medium, we mean a volume of liquid or a volume of liquid and gas.

For this, the invention allows to control an injected fluid into the volume of liquid cell culture medium VL and/or to control an extracted fluid from the volume of liquid cell culture medium VL. In particular, the invention allows to control the flow rate of the injected fluid and/or the extracted fluid and to control the temperature and the internal pressure inside the closed chamber, and a dissolved gas concentration and/or a pH of the volume of liquid cell culture medium VL.

Thus, the dissolved gas concentration and/or the pH of the volume of liquid cell culture medium VL in the altered pressure closed chamber can be measured and adjusted accordingly.

The invention is useful for conducting controlled biological assays in the fields of pharmacological and biotechnology discovery, controlled cell differentiation like diagnostic assays for personalized medicine, therapeutic and cosmetic assays, compound research and assays, and custom assays, as well as in all life science experiments requiring precise control of the environmental conditions of cells, tissues or organoids.

More particularly, the invention proposes to inject a fluid into the cell culture medium, and/or simultaneously to block or to enable the extraction of a portion of the cell culture medium to modify in real time the internal pressure inside said at least one closed chamber. The local pressure of the volume of liquid cell culture medium VL applied to said cultured biological entity is also modified as well as the dissolved gas concentration and/or the pH of the volume of liquid cell culture medium VL. The volume of liquid cell culture medium VL applies a pressure force (named local pressure) on the biological entity.

For example, the fluid that is injected in the closed chamber is, for example, a gas preferably chosen from among O₂, CO₂, N₂ or their mixtures, a mix of pre-filtered gasses (predetermined mix of oxygen, carbon dioxide (CO2), nitrogen), or a pre-conditioned cell media (such as EBM2 or EGM) following enrichment from pre-filtered gasses (oxygen, CO2 and Nitrogen).

This allows to mimic *in vivo* microphysiological conditions by controlling the flow rate of the injected and/or extracted fluid and controlling the internal pressure inside said at least one closed chamber (1), while simultaneously maintaining other critical chemical and physical parameters of the liquid cell culture medium. Furthermore, this allows to maintain the cell culture conditions over the long-term (i.e., between 1 day and 60 days) and to apply specific physiological or physio pathological conditions (for example pH, dissolved Oxygen concentration...etc.) to this cell culture in a time-dependent manner. In other words, the microfluidic device, fluidic device or fluidic assembly and cell culture system according to the invention allows to maintain a stable and time-dependent cell culture environment to mimic *in vivo* microphysiological conditions including: gas composition of the volume of liquid cell culture medium VL, internal pressure inside the closed chamber (1), pH of the volume of liquid cell culture medium VL (due to its well-known dependence on dissolved CO₂ in aqueous solutions) ...etc.

However, the cell culture of the invention could also be used in research to determine the effects and advantages of pressure / mechanotransduction on a specific biological entity. Hence, it is possible to then determine the most effective local pressure for the specific biological entity.

For this purpose, a predetermined local pressure and a predetermined dissolved gas concentration and/or a predetermined pH of the volume of liquid cell culture medium VL have to be predetermined as a function of the cultured biological entity.

By "predetermined", we mean in the following description values of pH and dissolved gasses in function of the local pressure that is applied on the biological entity.

This step of setting predetermined values could also be used in research to determine the effects and advantages of pressure/mechanotransduction on pre-designed cell models cell models, to then determine the most effective pressure for a specific model,

Subsequently, for the purposes of simplification, "biological entity" is understood to mean a non-organized or organized cell culture, namely a cell assembly such as for example tissues, spheroids or organoids. These cells can for example be from animals, such as humans, mice, or plants. Thus, the cultured biological entity is composed of at least one cell. Preferably, the cultured biological entity is composed of at least one cancer cell.

"Multi-well cell culture plate" is understood to mean standard plates comprising at least six wells 34, 35, or closed chambers, in which a specific nutrient culture medium, for example a volume of liquid cell culture medium, certain chemical or biochemical compounds, with the cell type studied is introduced, as well as cells to be cultured, like a cultured biological entity.

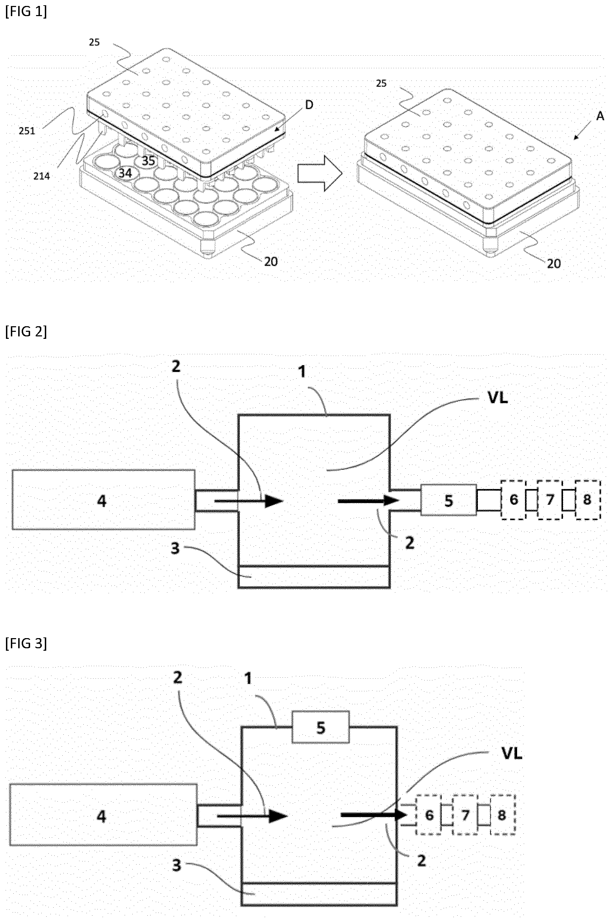

Thus, multiple closed chambers 34, 35 are formed as illustrated in figure 1, in which we can see a microfluidic device D with shape selected from among the cubic, rectangular shapes but preferably substantially parallelepiped-shaped comprises a cover, also called fluid-routing layer 25, and a part called the liquid-routing layer 21 (also called manifold 21), comprising a series of nozzles or orifices or ducts 214 forming the base of this microfluidic device D. The nozzles or orifices or ducts 214 carry the liquid into the multi-well cell culture plate 20 and out of it.

The fluids-routing layer 25 has, on two opposite edges, one or more connection orifice(s) or fluidic connection duct(s) 251, 255 for attaching at least one fluid connector (not shown) from a controller that is a fluid flow controller (shown in figures 2 to 5). The controller externally supplies a fluid to cell culture systems according to the invention. The controller controls and adjusts in real time at least the flow rate of the injected fluid and an internal pressure inside the closed chambers, or wells 34, 35. This fluids-routing layer 25 can be entirely opaque or, in a variant, allow the passage of light in order to obtain a real-time optical image of the cultured biological entities.

For reasons of clarity and ease of understanding, in what follows, as shown in Figures 2 to 5, we will concentrate on the implementation of the invention on only one closed chamber 1 that contains a volume of liquid cell culture medium VL that applies a pressure force, or local pressure, on the cultured biological entity 3.

This closed chamber 1 comprises an inlet side through which the fluid 2 is injected, and an outlet side through which the fluid 2, in particular a portion of the volume of liquid cell culture medium VL, is extracted.

The closed chamber 1 is connected to the microfluidic device D that comprises two microfluidic circuits.

However, it should be noted that the microfluidic device D can have more than two microfluidic circuits.

Moreover, in Figures 2 to 5, each microfluidic circuit comprises a single microfluidic channel, but it should be noted that each circuit can comprise several microfluidic channels in different configurations (for example one channel can separate to form two different channels, and conversely two channels can be joined together to form one.

The inlet side of the closed chamber 1 is connected to a microfluidic channel of a first microfluidic circuit of the microfluidic device. The outlet side of the closed chamber 1 is connected to a microfluidic channel of a second microfluidic circuit of the microfluidic device.

In each of the microfluidic channels, the injected fluid 2 and the extracted fluid 2 can circulate. In other words, each microfluidic circuit is dedicated to the circulation of either a gas or a liquid.

"Microfluidic channel" or "channel" is understood to mean channels with cross sections between 0.1µm² and 25mm².

The injection and the extraction could be carried on simultaneously or alternatively.

The inlet side for injecting fluid 2 is, for example, a fluid connection orifice or fluidic connection duct allowing the attachment of a fluid connector coming from the controller 4 that supplies the injected fluid 2. The controller 4 controls at least the flow rate of the injected fluid 2 and the internal pressure of the closed chamber 1 in real time. This control of pressure and/or flow rate values by at least one controller 4 can be done manually, i.e. an operator will modify the internal pressure and/or flow rate values. Alternatively, this control of the internal pressure and/or flow rate values by at least one controller 4 can be carried out automatically, i.e. without the operator's intervention.

The injecting and/or extracting a fluid 2 at a flow rate into and/or from said volume of liquid cell culture medium VL so that the fluid 2 moving within said volume of liquid cell culture medium VL resulting from the injection and/or the extraction involves a change in the local pressure of the volume of liquid cell culture medium VL applied to the cultured biological entity 3 inside the closed chamber 1 and a change in dissolved gas concentration and/or pH of the volume of liquid cell culture medium VL.

In figures 2 to 5, the controller 4 is configured to adjust the flow rate of the injected fluid 2 at less than or equal to 20 ml/min and the internal pressure inside the closed chamber 1 at less than or equal to 10 bar. The closed chamber 1 comprises the volume of liquid cell culture medium VL and the cultured biological entity 3.

For example, the controller 4 is a fluid pressure control or a mass flow control mechanism.

In a variant non-illustrated, the controller 4 can be a PID (Proportional-Integral-Derivative) controller that allows to compensate for pressure effects quickly and sensitively, bringing the closed chamber 1 to uniform conditions very close to the set values. The PID controller can compensate with precise, rapid temperature variations that occur due to pressure changes, to ensure the optimum biophysical parameters.

Preferably, the controller 4 contains a pressure sensor in order to measure the internal pressure inside the closed chamber 1.

Figure 2 shows a diagram of an example of an embodiment of a part of microfluidic device suitable for implementing the method according to the invention.

In the embodiment illustrated in figure 2, a pressure-release valve 5, that contained another pressure sensor, is connected to the outlet side of the closed chamber 1. This pressure-release valve 5 is actuated in synchronization with the controller 4 that contains a pressure sensor. This pressure-release valve 5 can facilitate the control of the portion of the volume of liquid cell cultured medium that is extracted notably in function of the flow rate of the injected fluid 2 and the measured internal pressure inside the closed chamber 1.

It is therefore possible to determine the local pressure at least on the basis of the measured internal pressure obtained by the pressure sensor. This determination is eventually carried out by the use of a determination unit implementing a specific algorithm adapted to calculate the local pressure based on the internal pressure. Preferably, the determination unit is connected to the controller 4 that is connected to a least the internal pressure sensor, and a dissolved gas concentration sensor 7 and/or a pH sensor 8.

Moreover, in this embodiment, the dissolved gas concentration sensor 7 and the pH sensor 8 are also connected to the outlet side of the closed chamber 1 and configured to collect measure(s) of the dissolved gas(es) concentration and/or the pH of the portion of the volume of liquid cell culture medium VL after the exchange of gas(es) molecules between the cell culture medium and the biological entity 3. Then, these sensors transmit the data to the controller 4. Thus, it is possible to measure the dissolved gas concentration and the pH of the volume of liquid cell culture medium VL during the extraction of a portion of the volume of liquid cell culture medium VL. In this way, it is possible to verify the dissolved gas concentration and pH of the volume of liquid cell culture medium VL after the exchange of molecules between the fluid 2 that is injected and the volume of liquid cell culture medium VL contained in the closed chamber 1.

Thus, this controller 4 can be configured to receive the data collected by the dissolved gas(es) concentration sensor 7 and/or the pH sensor 8, and also by the pressure sensor.

This dissolved gas concentration sensor 7 and this pH sensor 8 are also connected to the controller 4.

Hence, the control can adjust at least the flow rate of the injected fluid 2 and/or the extracted fluid 2 and the internal pressure so that the determined local pressure and the measured dissolved gas concentration and/or the measured pH of the volume of liquid cell culture medium VL correspond to the predetermined local pressure and the predetermined dissolved gas concentration and/or the predetermined pH of the volume of liquid cell culture medium VL respectively.

The microfluidic device of figure 2 further comprises a temperature sensor 6 configured to measure a temperature in said at least one closed chamber 1. Preferably, the measured temperature is transmitted to the controller 4 in order to consider the temperature for adjusting the flow rate of the injected and/or extracted fluid 2 and the internal pressure.

Preferably, the temperature sensor 6 is connected to a control unit itself connected to heating means surrounding closed chamber 1, for example a polyimide heating plate for multi-wells, configured to modify the temperature inside said at least one closed chamber 1 to maintain the temperature inside the closed chamber 1 at a temperature between 30°C and 42°C, preferably around 37°C. Thus, the PID controller further allows to easily adjust the temperature altered through internal pressure modification.

Also, based on the measured internal pressure, on the temperature within the closed chamber 1, and on measured dissolved gas concentration 8 and on the measured pH of the volume of liquid cell culture medium VL, it is possible to determine precisely the local pressure of the volume of liquid cell culture medium VL applied to said cultured biological.

Thus, by the use of the controller 4 and the pressure-release valve 5, the injected fluid 2 moves within the volume of liquid cell culture medium VL and a portion of the volume of liquid cell culture medium VL is extracted, this involving a change of the internal pressure inside the closed chamber 1 and a change in dissolved gas concentration and pH of the volume of liquid cell culture medium VL, thus a change in the local pressure.

Hence, in this embodiment, the controller 4 is configured to control the injection and the extraction of the fluid 2 into or from the closed chamber 1 in order to apply and maintain system pressure in conjunction with the pressure-release valve 5.

Preferably, this control is carried out with the use of a calculation unit configured to calculate the flow rate of the fluid 2 that is necessary to inject and the flow rate of the portion of the volume of liquid cell culture medium VL that is necessary to extract, and , and the internal pressure inside the closed chamber 1, so that the determined local pressure and the measured dissolved gas concentration and the measured pH of the volume of liquid cell culture medium VL correspond to the predetermined pressure and the predetermined dissolved gas concentration and the predetermined pH of the volume of liquid cell culture medium VL, respectively.

The calculation unit is further configured to allow the controller 4 to control a composition of the injected fluid 2 to readily alter the dissolved gas concentration and/or the pH of the volume of liquid cell culture medium VL for facilitating the correspondence of the step of adjusting between the measured dissolved gas concentration and/or the measured pH and the predetermined dissolved gas concentration and/or the predetermined pH respectively.

Preferably, the control of the composition and the pH of the injected fluid 2 uses a feed-back loop automated system based on a mathematical algorithm and/or an artificial intelligence module.

Preferably, the step of controlling the composition and/or the pH of the injected fluid 2 uses a feed-back loop automated system based on a mathematical algorithm and/or an artificial intelligence module connected to the calculation unit.

The calculation unit is thus configured to analyze and compare the measured dissolved gas concentration and/or the pH measurements to a predetermined dissolved gas concentration and/or pH value of the volume of liquid cell culture medium VL that is extracted and flows in contact with these dissolved gas concentration sensor 7 and pH sensor 8, along with the pressure sensors to ensure the predetermined internal pressure.

It should be noted that by adjusting CO₂ concentration of the liquid cell culture medium, its pH can be altered. Indeed, for any given partial pressure of a gas, solubility is inversely proportional to temperature in aqueous solutions and decreases at increased concentration of electrolytes. These can modify indirectly other parameters, for example, when CO₂ dissolves in water it creates carbonic acid (H₂CO₃) and the hydrogen ions present in carbonic acid make water acidic thus lowering the pH.

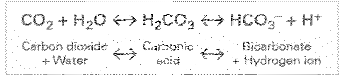

The calculation unit is connected to the controller 4.

Then, the controller 4, in conjunction with the pressure sensor and pressure-release valve 5, can condition the volume of liquid cell culture medium VL regarding the flow rates and internal pressures calculated by the calculation unit by adjusting the flow rate and the local pressure of the fluid 2 that is necessary to inject and the flow rate of the portion of the volume of liquid cell culture medium VL that is necessary to extract.

Advantageously, the step of adjusting the flow rate of the injected fluid 2 and/or the extracted fluid 2 and the internal pressure uses a feed-back loop automated system based on a mathematical algorithm and/or an artificial intelligence element considering the determined local pressure and the measured dissolved gas concentration and/or the measured pH of the volume of liquid cell culture medium.

Figure 3 shows a diagram of an example of an embodiment of a part of microfluidic device suitable for implementing the method according to the invention.

In the embodiment illustrated in figure 3, the pressure-release valve 5 is placed through a wall of the closed chamber 1. In this configuration, this provides a finer measure and therefore precisely control of the internal pressure within the closed chamber 1.

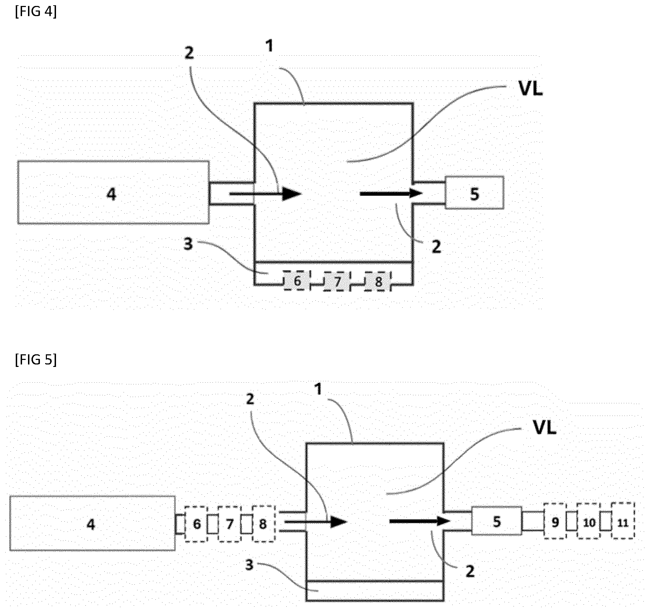

Figure 4 shows a diagram of an example of an embodiment of a part of microfluidic device suitable for implementing the method according to the invention.

In the embodiment illustrated in figure 4, the temperature sensor 6, the dissolved gas concentration sensor 7 and the pH sensor 8 are placed in the closed chamber 1. Preferably, these sensors are placed near the cultured biological entity 3, i.e. at a bottom of the closed chamber 1. Thus, a local detection of the volume of liquid cell medium within the closed chamber 1 results in more precise values of pH, DO and temp within the closed chamber 1 at the site of the biological entity 3. Also, these values are more accurate to determine the effective pressure of the volume of liquid cell culture medium VL applied to said cultured biological entity 3 through comparing the alteration of the pH, DO and temperature differences introduced by the local pressure change.

In another embodiment as illustrated in figure 5, the temperature sensor 6, the pH sensor 8 and the dissolved gas concentration sensor 7 are placed upstream, namely between the controller 4 and the closed chamber 1 in the first microfluidic circuit. In this configuration, the cell culture system further comprises a complementary temperature sensor 9 and/or a complementary pH sensor 11 and/or a complementary dissolved gas concentration 10 sensor placed downstream, namely in the second microfluidic circuit. Hence, the temperature, the pH and the dissolved gas sensors are used (6, 7, 8) in conjunction with complementary temperature, pH and dissolved gas sensors (9,10,11) to generate differential measurements of temperature, pH and dissolved gases in the closed chamber 1 and to detect and condition the volume of liquid cell culture medium VL (pH and/or dissolved gases) so that the effective pressure that applies the volume of liquid cell culture medium VL on the cultured biological entity 3 correspond to the predetermined by altering the local pressure and flow rate of the injected fluid 2 and the portion of the volume of liquid cell culture medium VL that is extracted. This configuration allows to ensure the correct liquid cell culture medium conditioning is present throughout the microfluidic device.

Moreover, in the embodiment of figure 5, the controller 4 is then able to compare the dissolved gas concentration and/or the pH value collected by the upstream sensors, with the dissolved gas(es) concentration and/or the pH value collected by the downstream sensors. Then this data is compared to the predetermined dissolved gas concentration and/or predetermined pH value (s) of the liquid cell culture medium.

By comparing the data collected by the downstream sensors with the predetermined dissolved gas concentration and/or predetermined pH value, or by comparing the data collected by the upstream and downstream sensors with the predetermined gas concentration and/or predetermined pH value(s), the controller 4 is able to adjust the different parameters (i.e. pressure(s) or partial pressure(s), fluid injection flow rate(s), local pressure, gas composition (i.e nature of the fluid)).

For example, the controller 4 is able to adjust the flow rate of the injected fluid 2, the local pressure, the composition of the injected fluid 2 and also the activation of the release-pressure valve 5.

Alternatively, the calculation unit processes the data as previously described, and it is configured to transfer the result to an element configured to display the result on a human-machine interface so that the operator manually adjust the fluid 2 flow rate and local pressure accordingly.

For this, the calculation unit implements a mathematical algorithm (e.g., proportional feedback closed-loop control, active disturbance rejection control (ADRC)) and/or an artificial intelligence module (e.g., neural network proportional-integral-derivative control) that enables to compare the measured data to the predetermined dissolved gas concentration and predetermined pH value, and predetermined pressure, in order to adjust the fluid 2 moving within the volume of liquid cell culture medium VL to obtain a stable pressure of the volume of liquid cell culture medium VL that corresponds to the predetermined pressure.

Thus, it is possible to achieve very precise local pressure applied by the volume of liquid cell culture medium VL on the cultured biological entity 3 that has been set to be guaranteed in time.

In a non-illustrated embodiment, rather than the pressure sensor being contained into the controller 4, the pressure sensor is connected to the first microfluidic circuit, for example between the controller 4 and the inlet of the closed chamber 1.

It is to note that intermittent injection and/or extraction of fluid 2 can also be used with slight fluctuations around the predetermined local pressure value that is set (for instance for a set value of 2 bar, for example pressure variations of 1.8 to 2 bar). These brief fluctuations result in low level fluidic injection and/or extraction meaning replenishment of the volume of liquid cell culture medium VL during long-term cell culture studies. Another advantage being avoidance of non-physiological static closed chamber 1 conditions (a central limitation of in-vitro cell models) by causing perturbations in the volume of liquid cell culture medium VL within the closed chamber 1.

Preferably, with these embodiments, it is possible to determine a physiological and/or a pathophysiological condition of the cultured biological entity 3 which presents as a result of a change in the phenotype of the cultured biological entity 3. In particular, this determination allows to determine a metastatic potential of the cancer cell.

In all embodiments, the approach can also be used with 2D cell culture but also more complex 3D models (such as spheroids). This can be achieved by using the spheroid wells (of 4DCell) to provide localized pressure across up to multiple spheroids within a single well. Also in all embodiments, flexible hydrogel bases can be used to better replicate the physiological response to pressure in comparison to the rigidity of the plastic multi-well plate.

Thus, advantageously, when the cell culture system comprises a plurality of closed chambers as illustrated in figure 1 for example, the step of measuring the internal pressure inside said each closed chamber corresponds to a step of calculating an average internal pressure based on the measured internal pressure inside each closed chamber, and the measured dissolved gas concentration and/or the measured pH of the volumes of liquid cell culture medium (VL) by at least pressure sensors, and dissolved gas concentration sensors and/or pH sensors.

Thanks to the invention, it is also possible to estimate, via the use of an algorithm, the internal pressure by following the fluctuation of the dissolved gas concentration and/or pH of the volume of liquid cell culture medium VL and the temperature inside the closed chamber 1. These estimations allow to maintain the effective local pressure to the predetermined local pressure.

## Claims

1. Method for controlling pressure and dissolved gas concentration and/or pH of a volume of liquid cell culture medium (VL) contained in at least one closed chamber (1) which comprises a cultured biological entity (3) composed of at least one cell, **characterized in that** the method comprises the following steps:
- setting a predetermined local pressure and a predetermined dissolved gas concentration and/or a predetermined pH to be satisfied by the volume of liquid cell culture medium (VL) as a function of the cultured biological entity (3),
- injecting and/or extracting a fluid (2) at a flow rate into and/or from said volume of liquid cell culture medium (VL) so that the fluid (2) moving within said volume of liquid cell culture medium (VL) resulting from the injection and/or the extraction involves a change in a local pressure of the volume of liquid cell culture medium (VL) applied to the cultured biological entity (3) inside said at least one closed chamber (1) and a change in dissolved gas concentration and/or pH of the volume of liquid cell culture medium (VL),
- measuring an internal pressure inside said at least one closed chamber (1), and a dissolved gas concentration and/or a pH of the volume of liquid cell culture medium (VL) by at least a pressure sensor, and a dissolved gas concentration sensor (7) and/or a pH sensor (8),
- determining the local pressure at least on the basis of a measured pressure obtained by the pressure sensor,
- adjusting, by a controller (4), at least the flow rate of the injected fluid (2) and/or the extracted fluid (2) and the internal pressure so that the determined local pressure and the measured dissolved gas concentration and/or the measured pH of the volume of liquid cell culture medium (VL) correspond to the predetermined local pressure and the predetermined dissolved gas concentration and/or the predetermined pH of the volume of liquid cell culture medium (VL) respectively,
during the step of adjusting, the flow rate of the injected fluid (2) and/or the extracted fluid (2) is less than or equal to 20 ml/min and the internal pressure is less than or equal to 10 bar.

2. The method according to claim 1, wherein said method further comprises a step of controlling a composition of the injected fluid (2) to readily alter the dissolved gas concentration and/or the pH of the volume of liquid cell culture medium (VL) for facilitating the correspondence of the step of adjusting between the measured dissolved gas concentration and/or the measured pH and the predetermined dissolved gas concentration and/or the predetermined pH respectively.

3. The method according to claim 2, wherein said step of controlling the composition and/or the pH of the injected fluid (2) uses a feed-back loop automated system based on a mathematical algorithm and/or an artificial intelligence module.

4. The method according to claims 1 to 3, wherein said step of adjusting the flow rate of the injected fluid (2) and/or the extracted fluid (2) and the internal pressure uses a feed-back loop automated system based on a mathematical algorithm and/or an artificial intelligence element considering the determined local pressure and the measured dissolved gas concentration and/or the measured pH of the volume of liquid cell culture medium (VL).

5. The method according to claims 1 to 4, wherein said method further comprises a step of determining a physiological and/or a pathophysiological condition of the cultured biological entity (3) which presents as a result of a change in the phenotype of the cultured biological entity (3).

6. The method according to claim 5, wherein when the cultured biological entity (3) is composed of at least one cancer cell, the step of determining a physiological and/or a pathophysiological condition of the cultured biological entity (3) corresponds to a step of determining a metastatic potential of said at least one cancer cell.

7. The method according to claims 1 to 6, wherein said method further comprises a step of measuring a temperature inside said at least one closed chamber (1) by at least a temperature sensor (6).

8. The method according to claims 1 to 7, wherein said at least one injected fluid (2) is chosen from among O₂, CO₂, N₂ or their mixtures.

9. The method according to claims 1 to 8, when the cell culture system comprises a plurality of closed chambers, the step of measuring the internal pressure inside said each closed chamber corresponds to a step of calculating an average internal pressure based on the measured internal pressure inside each closed chamber, and the measured dissolved gas concentration and/or the measured pH of the volumes of liquid cell culture medium (VL) by at least pressure sensors, and dissolved gas concentration sensors and/or pH sensors.

10. A cell culture system **characterized in that** it implements the method according to claims 1 to 9, and **in that** it comprises:
- at least one closed chamber (1) comprising a volume of liquid cell culture medium (VL) and containing a cultured biological entity (3), said at least one closed chamber (1) comprising, for injecting and/or extracting a fluid (2) into and/or from the volume of liquid cell culture medium (VL), an inlet side through which the fluid (2) is injected and an outlet side through which the fluid (2) is extracted to control the local pressure and dissolved gas concentration and/or pH of the volume of liquid cell culture medium (VL),
- a microfluidic device comprising:
∘ at least two microfluidic circuits:
▪ a first of said at least two microfluidic circuits comprising at least one microfluidic channel configured to be connected to the inlet side of said at least one closed chamber (1), and
▪ a second of said at least two microfluidic circuits comprising at least one microfluidic channel configured to be connected to the outlet side of said at least one closed chamber (1),
- a controller (4) connected to said at least one microfluidic channel of the first or the second of said at least two microfluidic circuits, the controller (4) being configured to adjust the flow rate of the injected and/or extracted fluid (2) and an internal pressure inside said at least one closed chamber (1),
- a pressure sensor configured to measure the internal pressure in said at least one closed chamber (1) and to transmit the measured internal pressure to the controller (4), the pressure sensor being connected to said at least one microfluidic channel of the first or the second of said at least two microfluidic circuits or being connected to the interior of said at least one closed chamber (1),
- a dissolved gas concentration sensor (7) and/or a pH sensor (8) configured to measure a dissolved gas concentration and/or a pH in said at least one closed chamber (1) and to transmit the measured dissolved gas concentration and/or the measured pH to the controller (4), the dissolved gas concentration sensor (7) and/or the pH sensor (8) being connected to said at least one microfluidic channel of the first or the second of said at least two microfluidic circuits or being connected to the interior of said at least one closed chamber (1).

11. The cell culture system according to claim 10, wherein the cell culture system further comprises a pressure-release valve (5) actuated in synchronization with said controller (4), the pressure-release valve (5) being connected to the second of said at least two microfluidic circuits when the pressure sensor is connected to the first of said at least two microfluidic circuits.

12. The cell culture system according to claim 11, wherein the pressure sensor is contained inside the controller (4) when the controller (4) is connected to said at least one microfluidic channel of the first of said at least two microfluidic circuits.

13. The cell culture system according to claim 10, wherein the controller (4) is connected to said at least one microfluidic channel of the first of said at least two microfluidic circuits, the pressure sensor and the pressure-release valve (5) are connected to the second of said at least two microfluidic circuits.

14. The cell culture system according to claims 10 to 13, wherein the dissolved gas concentration sensor (7) and/or the pH sensor (8) are connected to said at least one closed chamber (1).

15. The cell culture system according to claims 10 to 14, when the dissolved gas concentration (7) sensor and/or the pH sensor (8) are placed upstream, namely between the controller (4) connected to said at least one microfluidic channel of the first of said at least two microfluidic circuits and said at least one closed chamber (1), the cell culture system further comprises a complementary dissolved gas composition sensor (10) and/or a complementary pH sensor (11) placed downstream, namely connected to said at least one microfluidic channel of the second of said at least two microfluidic circuits.

16. The cell culture system according to claims 10 to 15, wherein the cell culture system further comprises a temperature sensor (6) configured to measure a temperature in said at least one closed chamber (1) and to transmit the measured temperature to the controller (4), the temperature sensor (6) being connected to said at least one microfluidic channel of the first or the second of said at least two microfluidic circuits or inside said at least one closed chamber (1).

17. The cell culture system according to claim 16, wherein the temperature sensor (6) is connected to a control unit itself connected to heating means configured to modify the temperature inside said at least one closed chamber (1) as a function of the measured temperature.
